# EUROPÄISCHE PATENTSCHRIFT

(11) **EP 1 621 883 B1**
(45) Veröffentlichungstag und Bekanntmachung des Hinweises auf die Patenterteilung: **02.04.2008**
(21) Anmeldenummer: 05014774.3
(22) Anmeldetag: 07.07.2005
(51) Int. Cl.: G01N 33/34, G01N 21/15

(54) **Materialbahn-Inspektionsanlage**
Surveying device for material web
Dispositif d'inspection d'une bande de matériau

(30) Priorität: 08.07.2004 DE 102004033096
(43) Veröffentlichungstag der Anmeldung: 01.02.2006
(73) Patentinhaber: LSC Process- und Laborsysteme GmbH, 56566 Neuwied (DE)
(72) Erfinder: Windheuser, Richard, 56566 Neuwied (DE)
(74) Vertreter: Englaender, Klaus

(56) Entgegenhaltungen:
- EP-A- 0 297 271
- EP-A- 0 974 828
- DE-A1- 4 421 050
- DE-A1- 10 004 612
- US-A- 4 913 049

## Beschreibung

Die vorliegende Erfindung betrifft Materialbahn-Inspektionsanlage, aufweisend ein Flachbett, in welchem Materialbahn-Inspektionselemente unter einer ebenen Schutzabdeckung angeordnet sind, über welche eine zu inspizierende Materialbahn hinwegbewegt wird, wobei seitlich am Flachbett über der Schutzabdeckung eine Einrichtung zum Blasen eines gasförmigen Mediums quer über die Schutzabdeckung angeordnet ist gemäß dem Oberbegriff des Anspruchs 1. Eine solche Anlage ist beispielsweise in der Druckschrift EP 0 297 271 A2 offenbart.

Materialbahn-Inspektionsanlagen dienen als Analysewerkzeug zur Produktivitätssteigerung bei beispielsweise der Pulpen-und Papierherstellung sowie beim Verarbeiten von Papierbahnen und dergleichen, wie etwa beim Bedrucken von Papierbahnen. Die hierzu benötigten Sensoren bzw. allgemein Materialbahninspektionselemente sind unter einer Schutzabdeckung beispielsweise in Gestalt einer Glasscheibe angeordnet, um sie vor der relativ hohen Temperatur und der starken Verschmutzung zu schützen, welchen die Inspektionselemente in der Praxis, beispielsweise bei der Papierherstellung ausgesetzt sind. Aufgrund des Verschmutzungsproblems kann es sogar vorkommen, dass ein Bandinspektionssystem bei der Produktion nicht einsetzbar ist.

Es existieren zwei Ansätze, dem Verschmutzungsproblem entgegenzuwirken. Gemäss einem ersten Ansatz wird zu Reinigungszwecken das Flachbett bzw. der so genannte Lichtbalken an die darüber geleitete Papierbahn in Kontakt mit dieser herangefahren, so dass die mit relativ hoher Geschwindigkeit bewegte Bahn die Schutzabdeckung des Flachbetts insofern reinigt, als Schmutz auf dieser Abdeckung von der Bahn mitgerissen wird.

Diese Flachbettreinigung durch mechanischen Kontakt mit der darüber hinweg bewegten Bahn hat jedoch zur Folge, dass die Bahn reißen oder beschädigt werden kann. Besonders kritisch ist ein derartiges Reißen bzw. eine Beschädigung der bahn bei der Produktion von Tissue. Gemäß dem zweiten Ansatz schlägt die EP-A- 0 784 093 eine Materialbahn-Inspektionsanlage mit den Merkmalen des Oberbegriffs des Anspruch 1 vor. Insbesondere offenbart diese Druckschrift eine Material-Inspektionsanlage, die eine Blaseinrichtung zum Entfernen von Schmutz aufweist, wobei ein gasförmiges Medium über ein längliches Rahmenteil der Schutzabdeckung und darin befindliche Kanäle oberhalb der Schutzabdeckung eingeleitet und über die Abdeckung geblasen wird. Diese Reinigungsart hat gegenüber derjenigen gemäß dem ersten Aspekt den Vorteil, dass die Gefahr einer Beschädigung der Bahn durch mechanische Einwirkung auf diese nur bei großen Bahngeschwindigkeiten besteht. Nachteilig an diesem bekannten Reinigungskonzept ist jedoch, dass es nicht flächendeckend und für extreme Verschmutzungen nicht hinreichend wirkt.

Es besteht deshalb ein Bedarf auf diesem Gebiet der Technik, ein Mittel bereit zu stellen, das geeignet ist, extreme Verschmutzungsprobleme in den Griff zu bekommen.

Eine Aufgabe der vorliegenden Erfindung besteht deshalb darin, eine Materialbahn-Inspektionsanlage so auszugestalten, dass auch extreme Verschmutzungen des Flachbetts flächendeckend ohne Beschädigung der Bahn beseitigt werden.

Gelöst wird diese Aufgabe durch die Merkmale des Anspruchs 1. Vorteilhafte Weiterbildungen der Erfindung sind in den Unteransprüchen angegeben.

Erfindungsgemäss ist demnach vorgesehen, dass die Blas- bzw. Düseneinrichtung der Materialbahn-Inspektionsanlage einen an die Flachbahn anstellbaren bzw. an dieser montierbaren balkenförmigen Grundkörper mit einer von der Schutzabdeckung des Flachbettes abgewandten, zur Unterseite der Materialbahn weisenden Oberseite aufweist, wobei diese Oberseite eine Oberflächenstruktur derart aufweist, dass die Materialbahn unter Ausnützung des hydrodynamischen Paradoxons (Bernoulli-Effekt) an die Oberseite der Schutzabdeckung gesaugt wird, ohne diese Oberfläche zu berühren. Ferner umfaßt die Oberflächenstruktur gegenseitig beabstandete Querrillen, die sich zwischen den Längsrändern des Grundkörpers erstrecken und in diese ausmünden. Hierdurch wird zuverlässig vermieden, dass die Bahn mechanisch in Kontakt mit der Schutzabdeckung des Flachbetts gelangt, wodurch damit verbundene und beim Stand der Technik auftretende Probleme, wie Reissen der Bahn auch bei hohen Bahngeschwindigkeiten vermieden werden. Ausserdem eignet sich die Reinigungsart auch zur Beseitigung starker Bahnverschmutzung.

Aus der DE-A-100 04 612 ist eine Vorrichtung bekannt, bei der das aerodynamische Paradoxon ausgenutzt wird, um zugunsten einer präzisen Messung zu einem stabilen Zustand zwischen der Materialbahn aus Papier oder Pappe und der Messeinrichtung zu kommen, wodurch Abstandsschwankungen verringert werden. Bei dieser Vorrichtung wird das gasförmige Medium von oben auf die Materialbahn, deren Lage stabilisierend, geblasen. Zur Reinigung des unter der Materialbahn liegenden Flachbettes einer Materialbahn-Inspektionsanlage eignet sich das aerodynamische Paradoxon, so wie gemäss der Lehre in dieser Druckschrift eingesetzt wird, jedoch nicht.

Vorteilhafterweise ist die Blaseinrichtung so ausgestaltet, dass mit ihr gasförmiges Medium möglichst flächendeckend quer über die Flachbett-Schutzabdeckung geblasen werden kann. Zu diesem Zweck ist die Blaseinrichtung bevorzugt als Düseneinrichtung gebildet.

Die Düseneinrichtung umfasst bevorzugt mehrere Düsen, die gegenseitig beabstandet entlang dem Flachbett angeordnet sind.

Das erfindungsgemäße Ansaugen der Materialbahn in Richtung auf die Flachbett-Schutzabdeckung, ohne dass es zu einer Berührung zwischen Materialbahn und Schutzabdeckung kommt, ist vorteilhafterweise vorgesehen, dass die Oberflächenstruktur der Oberseite des balkenförmigen Grundkörpers gegenseitig beabstandete Querrillen umfasst, die sich zwischen den Längsrändern des Grundkörpers erstrecken und in diese ausmünden, so dass das mittels der Düseneinrichtung über die Schutzabdeckung des Flachbetts geblasene gasförmige Medium zwischen der Unterseite der Materialbahn und der kerbstrukturierten Oberseite des Grundkörpers austreten kann. Es hat sich herausgestellt, dass der angestrebte Bernoulli-Effekt besonders wirksam realisierbar ist, wenn die Querrillen in Gestalt von Kerben mit V-förmigem Querschnitt gebildet sind.

Das Flachbett ist herkömmlicherweise mit seitlichen Wülsten gebildet. Für diesen Fall ist der Grundkörper zum Übergreifen der seitlichen Wulst ausgebildet, beispielsweise dadurch, dass der Grundkörper der Form der Wulst folgt, und die Düse bzw. die Düsen sind im übergreifenden Grundkörperabschnitt gebildet.

Um die mehreren Düsen der Düseneinrichtung in wirtschaftlicher Weise mit gasförmigem Medium zu versorgen, sind diese eingangsseitig über einen gemeinsamen Zuführkanal mit einem Anschluss für das gasförmige Medium verbunden, welcher Anschluss an den Grundkörper gebildet ist.

Die Erfindung betrifft außerdem eine Vorrichtung zum Inspizieren einer Materialbahn, insbesondere einer Papierbahn, mit einem an der Materialbahn positionierbaren Messwagen, der die erfindungsgemäße und wie vorstehend angeführt ausgebildete Materialbahn-Inspektionsanlage umfasst.

Schließlich betrifft die Erfindung außerdem eine Vorrichtung zum Inspizieren einer Materialbahn, insbesondere eine Papierbahn, mit der erfindungsgemäßen Materialbahninspektionsanlage, die vorstehend erläutert ist, welche Anlage an die Materialbahn in eine Materialbahn-Verarbeitungsanlage integriert ist.

Nachfolgend wird die Erfindung anhand der Zeichnung beispielhaft näher erläutert; in dieser zeigen:

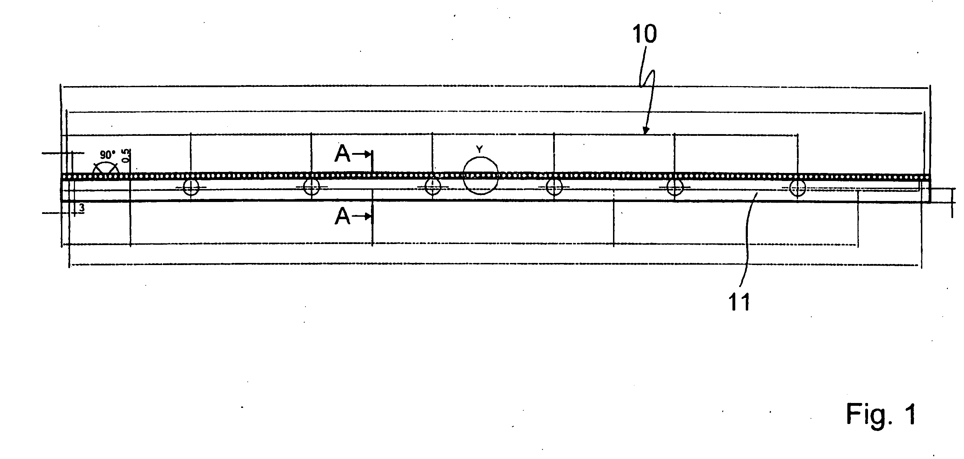

Fig. 1 eine Rückseitenansicht der Düseneinrichtung der erfindungsgemäßen Materialbahn-Inspektionsanlage,
Fig. 2 eine vergrößerte Detailansicht einer mit dem Kreis Y bezeichneten Einzelheit von Fig. 1,
Fig. 3 eine Schnittansicht entlang A-A in Fig. 1 in Pfeilrichtung gesehen, und
Fig. 4 eine Stirnseitenansicht der Düseneinrichtung von Fig. 1.

Die in den Figuren in verschiedenen Ansichten gezeigte Düseneinrichtung dient zum Blasen von einem gasförmigen Medium, bevorzugt Druckluft, quer über eine nicht gezeigte Schutzabdeckung eines nicht gezeigten Flachbetts einer Materialbahn-Inspektionsanlage, um sich dort ansammelnde Verschmutzung zu entfernen.

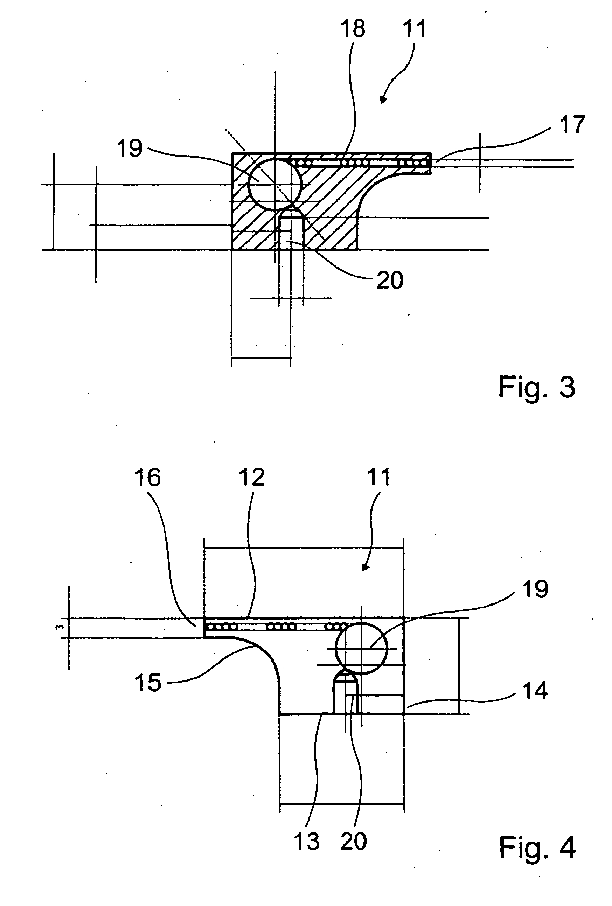

Im Einzelnen umfasst die Düseneinrichtung 10 einen balkenförmigen Grundkörper 11 mit der aus Fig. 3 und 4 hervorgehenden Querschnittsform. Wie aus Fig. 3 und 4 hervorgeht, weist der Grundkörper 11 eine ebene Unterseite 12, eine dazu parallel verlaufende ebene Oberseite 13, eine Unterseite 12 und Oberseite 13 verbindende ebene Außenseite 14 und eine konvex gekrümmte Innenseite 15 auf, die sich unmittelbar an die Unterseite 12 anschließt und über eine schmale Düsenauslassseite 16 in die Oberseite 13 übergeht. Die Düsenauslassseite 16 verläuft im Wesentlichen allgemein parallel zur Außenseite 14 und enthält eine Vielzahl von Düsenöffnungen 17 von Düsen, die in Längsrichtung bevorzugt gleichmäßig beabstandet im Grundkörper 11 gebildet sind. Die Düsenöffnungen 17 bilden Teil einer Mehrzahl von Düsen, die außerdem jeweils einen Düsenkanal 18 umfassen, der sich ausgehend von der Düsenöffnung 17 einwärts in den Grundkörper hinein erstreckt sowie knapp unterhalb der Oberseite 13 des Grundkörpers 11 sowie parallel zu dieser Seite verläuft. Mit dem innenliegenden Ende mündet der jeweilige Düsenkanal 18 in einen Zuführkanal 19 für Druckluft, der sich über die gesamte Länge des Grundkörpers 11 erstreckt und an einer Stirnseite des Grundkörpers 11 in einen nicht gezeigten Anschluss für Druckluft mündet. An der anderen Stirnseite des Grundkörpers 11 ist der Zuführkanal 19 bevorzugt verschlossen.

Zur Befestigung des Grundkörpers 11 an der seitlichen Peripherie eines Flachbetts der Materialbahn-Inspektionsanlage sind in den Grundkörper, in dessen Unterseite 12 mündend, mehrere Bohrungen 20 vorgesehen, die bevorzugt als Gewindebohrungen gebildet sind. Wenn der Grundkörper 11 bestimmungsgemäß am Flachbett montiert ist, übergreift der Grundkörper 11 mit seiner konkav gekrümmten Innenseite 15 eine dort üblicherweise vorgesehene Gummiwulst, so dass die Düsenöffnungen 17 oberhalb dieser Wulst zu liegen kommen, so dass Druckluft über die Düsen 17 quer über die nicht gezeigte Schutzabdeckung des Flachbetts geblasen werden kann, um bei der Produktion dort anfallende Verschmutzungen vom Flachbett herunter zu blasen.

Um zu verhindern, dass die über das Flachbett bewegte, zu inspizierende Bahn durch die über die Düsen ausgeblasene Druckluftströmung auf die Schutzabdeckung des Flachbetts gesaugt wird und damit in die Bahn beschädigenden mechanischen Kontakt mit dieser Schutzabdeckung gelangt, ist die Oberseite 13 des Grundkörpers 11 der Düseneinrichtung 10 mit einer Oberflächenstruktur derart versehen, dass die Materialbahn unter Ausnützung des hydrodynamischen Paradoxons (Bernoulli-Effekt) zwar auf die Oberseite der Schutzabdeckung gesaugt wird, diese Oberfläche jedoch nicht berührt.

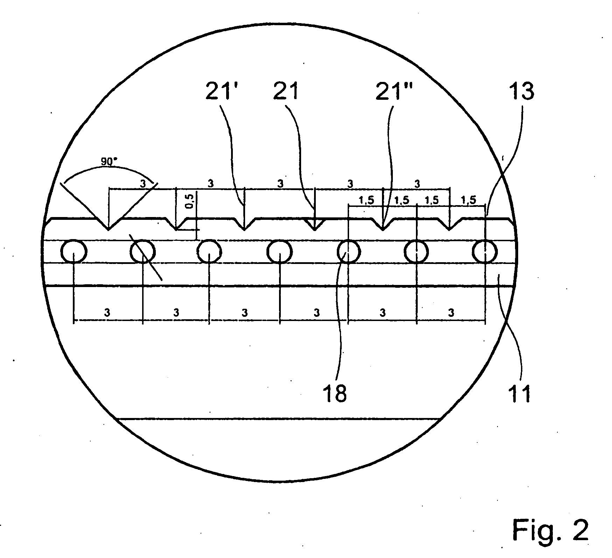

Eine entsprechende Oberflächenstruktur geht aus Fig. 2 hervor. Demnach umfasst diese Oberflächenstruktur auf der Oberseite 13 des Grundkörpers 11 eine Vielzahl von gegenseitig beabstandeten Querrillen in Gestalt von Kerben 21 V-förmigen Querschnitts. In Fig. 2 sind drei nebeneinander beabstandet angeordnete Kerben mit 21, 21' und 21" bezeichnet. Bevorzugt schließen die Flanken der Kerben 21 einen Winkel von etwa 90° ein. Ein bevorzugter gegenseitiger Abstand beträgt 3 mm und der bevorzugte Abstandsbereich liegt zwischen 1 mm und 10 mm. Die bevorzugte Tiefe der Kerben beträgt 0,5 mm und der Tiefenbereich für die Kerben 21 liegt zwischen 0,1 und 1,0 mm. Bevorzugt sind die Bereiche der gekerbten Oberseite 13 zwischen den Kerben 21 glatt und eben gebildet. Eine Alternative stellt eine leicht konkave Krümmung dieser Zwischenbereiche dar.

Aus Fig. 2 geht außerdem die Zuordnung der Düsenkanäle 18 zu den Kerben 21 hervor. Demnach sind die Düsenöffnungen 17 mit gleichem gegenseitigen Abstand angeordnet wie die Kerben 21. Die Düsenkanäle 18 sind jedoch jeweils um einen halben gegenseitigen Abstand in Bezug auf die Kerben 21 versetzt angeordnet. Die Düsenöffnungen 17 besitzen bezogen auf die genannten Abmessungen der Düsenkanäle 18 bevorzugt einen Durchmesser von 0,1 mm (0,05 bis 1 mm) und die Länge der Düsenkanäle 18 beträgt bevorzugt ca. 30 mm (5 bis 50 mm). Ein bevorzugter Durchmesser für den Zuführkanal 19 bei den genannten übrigen Düsenabmessungen beträgt 8 mm (2 bis 15 mm).

Mit der vorstehend erläuterten Düseneinrichtung 10 wird erreicht, dass die Schutzabdeckung des Flachbetts zumindest im Wesentlichen flächendeckend mit Druckluft überstrichen wird, so dass eine Schmutzablagerung auf der Schutzabdeckung des Flachbetts auch in stark verschmutzen Umgebungen bei der Materialbahnproduktion und -verarbeitung zuverlässig unterbunden wird.

### Bezugszeichenliste

- 10: Düseneinrichtung
- 11: Grundkörper
- 12: Unterseite
- 13: Oberseite
- 14: Außenseite
- 15: Innenseite
- 16: Düsenauslassseite
- 17: Düsenöffnung
- 18: Düsenkanal
- 19: Zuführkanal
- 20: Bohrung
- 21: Kerben

## Patentansprüche

1. Materialbahn-Inspektionsanlage, aufweisend ein Flachbett, in welchem Materialbahn-Inspektionselemente unter einer ebenen Schutzabdeckung angeordnet sind, über welche eine zu inspizierende Materialbahn hinwegbewegt wird, wobei seitlich am Flachbett über der Schutzabdeckung eine Einrichtung (10) zum Blasen eines gasförmigen Mediums quer über die Schutzabdeckung angeordnet ist, wobei die Blas- bzw. Düseneinrichtung (10) einen balkenförmigen Grundkörper (11) mit einer von der Schutzabdeckung des Flachbettes abgewandten, zur Unterseite der Materialbahn weisenden Oberseite (13) umfasst, wobei diese Oberseite (13) eine Oberflächenstruktur derart aufweist, dass die Materialbahn unter Ausnutzung des hydrodynamischen Paradoxons (Bernoulli-Effekt) auf die Oberseite der Schutzabdeckung gesaugt wird, ohne diese Oberfläche zu berühren, **dadurch gekennzeichnet, daß** die Oberflächenstruktur gegenseitig beabstandete Querrillen (21) umfaßt, die sich zwischen den Längsrändern des Grundkörpers (11) erstrecken und in diese ausmünden.

2. Anlage nach Anspruch 1, **dadurch gekennzeichnet, dass** die Blaseinrichtung eine Düseneinrichtung (10) ist.

3. Anlage nach Anspruch 2, **dadurch gekennzeichnet, dass** die Düseneinrichtung (10) mehrere Düsen (17, 18) umfasst, die gegenseitig beabstandet entlang dem Flachbett angeordnet sind, um dessen Schutzabdeckung zumindest im Wesentlichen flächendeckend zu überstreichen.

4. Anlage nach Anspruch 1, 2 oder 3, **dadurch gekennzeichnet, dass** die Oberseite (13) des Grundkörpers (11) plan sowie parallel zur planen Schutzabdeckung des Flachbettes verläuft.

5. Anlage nach einem der Ansprüche 1 bis 4, **dadurch gekennzeichnet, dass** das mittels der Düseneinrichtung (10) über die Schutzabdeckung des Flachbettes ausgetragene gasförmige Medium zwischen der Unterseite der Materialbahn und der kerbstrukturierten Oberseite (13) des Grundkörpers (11) austretbar ist.

6. Anlage nach Anspruch 5, **dadurch gekennzeichnet, dass** die Querrillen Kerben (21) mit V-förmigem Querschnitt sind.

7. Anlage nach einem der Ansprüche 1 bis 6, **dadurch gekennzeichnet, dass** der Grundkörper (11) mit seiner Innenseite (15) eine seitliche Wulst des Flachbettes übergreift, wobei die Düse bzw. die Düsen (17, 18) im übergreifenden Grundkörperabschnitt gebildet ist bzw. sind.

8. Anlage nach einem der Ansprüche 2 bis 7, **dadurch gekennzeichnet, dass** die mehreren Düsen (17, 18) der Düseneinrichtung (10) eingangsseitig über einen gemeinsamen Zuführkanal (19) mit einem Anschluss für das gasförmige Medium am Grundkörper (10) verbunden sind.

9. Anlage nach einem der Ansprüche 1 bis 8, **dadurch gekennzeichnet, dass** das gasförmige Medium Druckluft ist.

10. Vorrichtung zum Inspizieren einer Materialbahn, insbesondere einer Papierbahn, mit einem an der Materialbahn positionierbaren Messwagen, der die Anlage nach einem der Ansprüche 1 bis 9 umfasst.

11. Vorrichtung zum Inspizieren einer Materialbahn, insbesondere einer Papierbahn, mit der Anlage nach einem der Ansprüche 1 bis 9, die in eine Materialbahn-Verarbeitungsanlage integriert ist.

## Claims

1. Material web inspection assembly, having a flat bed in which material web inspection elements are arranged under a flat protective cover, over which a material web to be inspected is moved, an apparatus (10) for blowing a gaseous medium transversely over the protective cover being arranged at the side of the flat bed, wherein the blowing or nozzle apparatus (10) comprises a bar-like base body (11) with an upper side (13) facing away from the protective cover of the flat bed and pointing towards the underside of the material web, this upper side (13) having a surface structure in such a way that the material web is attracted by suction to the upper side of the protective cover by utilizing the hydrodynamic paradox (Bernouilli effect) without touching this surface, **characterized in that** the surface structure comprises mutually spaced transverse grooves (21), which extend between the longitudinal edges of the base body (11) and open into the latter.

2. Assembly according to Claim 1, **characterized in that** the blowing apparatus is a nozzle apparatus (10).

3. Assembly according to Claim 2, **characterized in that** the nozzle apparatus (10) comprises a plurality of nozzles (17, 18), which are arranged spaced apart from one another along the flat bed in order to sweep over its protective cover in a manner at least substantially covering the area.

4. Assembly according to Claim 1, 2 or 3, **characterized in that** the upper side (13) of the base body (11) is planar and runs parallel to the planar protective cover of the flat bed.

5. Assembly according to one of Claims 1 to 4, **characterized in that** the gaseous medium discharged over the protective cover of the flat bed by means of the nozzle apparatus (10) is able to emerge between the underside of the material web and the notch-like structured upper side (13) of the base body (11).

6. Assembly according to Claim 5, **characterized in that** the transverse grooves are notches (21) with a V-shaped cross section.

7. Assembly according to one of Claims 1 to 6, **characterized in that** the inner side (15) of the base body (11) reaches over a lateral bead of the flat bed, the nozzle or the nozzles (17, 18) being formed in the overlapping base body section.

8. Assembly according to one of Claims 2 to 7, **characterized in that** the plurality of nozzles (17, 18) of the nozzle apparatus (10) are connected on the inlet side, via a common feed duct (19), to a connection for the gaseous medium on the base body (10).

9. Assembly according to one of Claims 1 to 8, **characterized in that** the gaseous medium is compressed air.

10. Apparatus for inspecting a material web, in particular a paper web, having a measuring carriage which can be positioned on the material web and which comprises the assembly according to one of Claims 1 to 9.

11. Apparatus for inspecting a material web, in particular a paper web, having the assembly according to one of Claims 1 to 9, which is integrated into a material web processing system.

## Revendications

1. Installation de contrôle d'une bande de matériau, comportant une table plate, dans laquelle des éléments de contrôle d'une bande de matériau sont disposés en dessous d'un cache de protection plan, sur lequel défile une bande de matériau à contrôler, un dispositif (10), destiné à souffler un fluide gazeux transversalement au-dessus du cache de protection, étant monté sur le côté de la table plate au-dessus du cache de protection, le dispositif de soufflage ou dispositif à buses (10) comportant un corps de base (11) en forme de barre avec une face supérieure (13), tournée en éloignement du cache de protection de la table plate et orientée vers la face inférieure de la bande de matériau, ladite face supérieure (13) étant munie d'une structure superficielle de telle sorte que, sous l'effet du paradoxe hydrodynamique (effet de Bernoulli), la bande de matériau est aspirée sur la face supérieure du cache de protection, sans entrer en contact avec cette surface, **caractérisée en ce que** la structure superficielle comporte des gorges transversales (21), qui sont ménagées à distance les unes des autres et qui s'étendent entre les bords longitudinaux du corps de base (11) et débouchent dans ceux-ci.

2. Installation selon la revendication 1, **caractérisée en ce que** le dispositif de soufflage est un dispositif à buses (10).

3. Installation selon la revendication 2, **caractérisée en ce que** le dispositif à buses (10) comporte plusieurs buses (17, 18), qui sont disposées à distance les unes des autres le long de la table plate, afin de balayer en couvrant au moins sensiblement la surface le cache de protection de la table plate.

4. Installation selon la revendication 1, 2 ou 3, **caractérisée en ce que** la face supérieure (13) du corps de base (11) est plane et est parallèle au cache de protection plan de la table plate.

5. Installation selon l'une quelconque des revendications 1 à 4, **caractérisée en ce que** le fluide gazeux, projeté au moyen du dispositif de buses (10) sur le cache de protection de la table plate, est apte à sortir entre la face inférieure de la bande de matériau et la face supérieure (13), structurée en gorges, du corps de base (11).

6. Installation selon la revendication 5, **caractérisée en ce que** les gorges transversales sont des entailles (21) avec une section en forme de V.

7. Installation selon l'une quelconque des revendications 1 à 6, **caractérisée en ce que** le corps de base (11) s'engage avec sa face intérieure (15) au-dessus d'un bourrelet latéral de la table plate, la buse et/ou les buses (17, 18) étant formée(s) dans la partie du corps de base couvrant ledit bourrelet latéral.

8. Installation selon l'une quelconque des revendications 2 à 7, **caractérisée en ce que** la pluralité de buses (17, 18) du dispositif à buses (10) est reliée du côté admission, via un canal d'admission (19) commun, à un branchement pour le fluide gazeux sur le corps de base (10).

9. Installation selon l'une quelconque des revendications 1 à 8, **caractérisée en ce que** le fluide gazeux est de l'air comprimé.

10. Dispositif de contrôle d'une bande de matériau, en particulier d'une bande de papier, comportant un chariot de mesure, qui peut être positionné sur la bande de matériau et qui comporte l'installation selon l'une quelconque des revendications 1 à 9.

11. Dispositif de contrôle d'une bande de matériau, en particulier d'une bande de papier, comportant l'installation selon l'une quelconque des revendications 1 à 9, qui est intégrée dans une installation de traitement d'une bande de matériau.
